# EUROPEAN PATENT SPECIFICATION

(11) **EP 3 311 734 B1**
(45) Date of publication and mention of the grant of the patent: **03.03.2021**
(21) Application number: 15879270.5
(22) Date of filing: 30.10.2015
(51) Int. Cl.: A61B 5/00, B63C 9/15, B63C 9/08, G08B 21/04, G08B 21/08

(54) **SWIMMING WEARABLE DEVICE AND UPPER LIMB WEARABLE DEVICE**
SCHWIMMENDE TRAGBARE VORRICHTUNG UND TRAGBARE VORRICHTUNG FÜR OBERE EXTREMITÄTEN
DISPOSITIF PORTABLE DE NATATION ET DISPOSITIF PORTABLE DE MEMBRE SUPÉRIEUR

(30) Priority: 18.06.2015 CN 201510341563
(43) Date of publication of application: 25.04.2018
(73) Proprietor: BOE Technology Group Co., Ltd., Beijing 100015 (CN); BOE Optical Science And Technology Co., Ltd., Suzhou, Jiangsu 215021 (CN)
(72) Inventor: ZHOU, Feng, Beijing 100176 (CN); CAO, Chunlei, Beijing 100176 (CN)
(74) Representative: Brötz, Helmut
(86) International application number: PCT/CN2015/093399
(87) International publication number: WO 2016/201859

(56) References cited:
- CN-A- 103 149 859
- CN-U- 204 021 227
- CN-U- 204 701 758
- CN-Y- 201 430 943
- FR-B1- 2 741 853
- TW-A- 200 832 292
- US-A1- 2007 123 121
- US-A1- 2008 266 118
- US-A1- 2010 298 899

## Description

### TECHNICAL FIELD

Embodiments of the present disclosure relate to a swimming wearable device and an upper limb wearable device.

### BACKGROUND

When swimming, leg or foot cramps (also called as muscle spasm) are main causes of drowning. How to enhance safety of swimming exercise and reduce drowning accidents becomes a technical problem to be solved at present. US20070123121A1 discloses a water safety device including a rapidly inflatable personal floatation device, a sensing and control unit, and an inflation device. US20100298899A1 discloses a wearable therapeutic device including subject medical condition sensors and subject activity sensors.

### SUMMARY

One of the objects of the embodiments of the present disclosure is to provide a swimming wearable device and an upper limb wearable device so as to improve safety of swimming exercise and reduce drowning accidents.

At least one embodiment of the present disclosure provides a swimming wearable device, comprising an upper body wearable device and a lower limb wearable device, wherein
the upper body wearable device comprises an electronic airbag, the lower limb wearable device comprises a muscle condition monitoring device; the muscle condition monitoring device is connected by signal with the upper body wearable device, and when a muscle spasm in lower limb is detected, the muscle condition monitoring device activates the electronic airbag to open.

When a swimmer wearing the swimming wearable device according to the embodiments of the present disclosure swims, the muscle condition monitoring device will send a signal to the upper body wearable device if it detects a spasm in leg or foot, and the upper body wearable device activates the electronic airbag to open. The electronic airbag in an open state can provide certain buoyancy to the swimmer and protect safety of the swimmer. Therefore, the swimming wearable device according to the embodiments of the present disclosure can enhance safety of swimming exercise and reduce drowning accidents.

In one embodiment of the present disclosure, the upper body wearable device comprises a vest-like upper body wearable device, a bandage-like upper body wearable device, a sticking upper body wearable device or an arm wearing upper body wearable device.

In one embodiment of the present disclosure, the lower limb wearable device comprises an ankle wearing lower limb wearable device, a bandage-like lower limb wearable device or a sticking lower limb wearable device.

In one embodiment of the present disclosure, the swimming wearable device comprises at least two lower limb wearable device and at least one upper limb wearable device, wherein
each upper limb wearable device comprises an electronic airbag and the muscle condition monitoring device of each of the at least two lower limb wearable devices is connected by signal with each of the at least one upper limb wearable device or the electronic airbag of one of the at least one upper limb wearable device, and when detecting a muscle spasm, the muscle condition monitoring device sends a signal to one of the at least one upper limb wearable devices or to the electronic airbag of one of the at least two upper limb wearable devices, so as to make the one of the at least one upper limb wearable devices activate the electronic airbag to open or to activate the electronic airbag to open.

When a swimmer wearing the swimming wearable device according to the embodiments of the present invention swims, the muscle condition monitoring device will send a signal to the upper limb wearable device if a spasm in leg or foot occurs. In response to the signal, the upper limb wearable device can activate the electronic airbag to open. The opened electronic airbag provides certain buoyancy for the swimmer and protect safety of the swimmer.

In one embodiment of the present disclosure, the lower limb wearable device further comprises an alarming device which is connected by signal with the muscle condition monitoring device. And when detecting a muscle spasm, the muscle condition monitoring device sends a signal to the alarming device to activate the alarming device to give an alarm. The alarm signal of the alarming device can notify the dangerous situation of the swimmer to others or a lifeguard, thereby reducing drowning accidents and further improving the swimmer's safety.

In one embodiment of the present disclosure, the muscle condition monitoring device of each lower limb wearable device is connected by wireless signal with the at least one upper limb wearable device or with the electronic airbag of the at least one upper limb wearable device. Connection by wireless signal between the muscle condition monitoring device and the upper limb wearable device can reduce binding sense of the swimmer and improve his/her comfort.

In one embodiment of the present disclosure, the upper body wearable device further comprises a display device. The swimmer can be aware of sport information conveniently and timely by observing display information of the display device.

In one embodiment of the present disclosure, at least one of a clock module, a timer module and a mileage calculating module is integrated in the display device.

In one embodiment of the present disclosure, the mileage calculating module comprises a GPS mileage calculating module or a vibration counting mileage calculating module.

### BRIEF DESCRIPTION OF THE DRAWINGS

In order to clearly illustrate the technical solutions of the embodiments of the disclosure, the drawings of the embodiments will be briefly described in the following; it is obvious that the drawings described below are only related to some embodiments of the disclosure and thus are not limitative of the disclosure.
Fig.1 is an illustrative structural view of a swimming wearable device according to an embodiment of the present disclosure;
Fig.2 is an illustrative structural view of a swimming wearable device according to another embodiment of the present disclosure;
Fig.3 is an illustrative structural view of a swimming wearable device according to yet another embodiment of the present disclosure;
Fig.4 is an illustrative structural view of an upper body wearable device and a lower limb wearable device according to an embodiment of the present disclosure;
Fig.5 is an illustrative structural view of an upper body wearable device according to an embodiment of the present disclosure; and
Fig.6 is an illustrative structural view of a lower limb wearable device according to an embodiment of the present disclosure.

### DETAILED DESCRIPTION

In order to make objects, technical details and advantages of the embodiments of the disclosure apparent, the technical solutions of the embodiment will be described in a clearly and fully understandable way in connection with the drawings related to the embodiments of the disclosure. It is obvious that the described embodiments are just a part but not all of the embodiments of the disclosure. Based on the described embodiments herein, those skilled in the art can obtain other embodiment(s), without any inventive work, which should be within the scope of the disclosure.

To reduce drowning accidents and improve safety of swimming exercise, embodiments of the present invention provide a swimming wearable device and an upper limb wearable device.

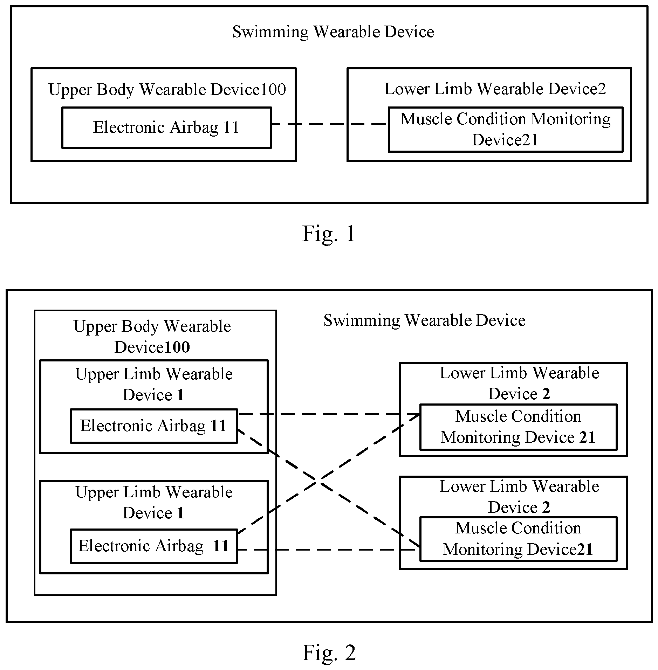

As illustrated in Fig.1, at least one embodiment of the present disclosure provides a swimming wearable device comprising an upper body wearable device 100 and a lower limb wearable device 2, wherein:

The upper body wearable device 100 comprises an electronic airbag 11. The lower limb wearable device 2 comprises a muscle condition monitoring device 21. The muscle condition monitoring device 21 is connected with the upper body wearable device 100 by signal or is directly connected with the electronic airbag 11 by signal. When the muscle condition monitoring device 21 detects muscle spasm in lower limb, the muscle condition monitoring device 21 sends a signal to the upper body wearable device 100 or the electronic airbag 11, with which it is connected by signal, so that the electronic airbag 11 is activated to open by the upper body wearable device 100 or the electronic airbag 11 is activated to open by the signal directly.

Forms of the upper body wearable device 100 and the lower limb wearable device 2 are not restricted. For example, the upper body wearable device 100 can be a vest-like upper body wearable device, a bandage-like upper body wearable device, a sticking upper body wearable device or an arm wearing upper body wearable device and etc.. The lower limb wearable device 2 can be an ankle wearing lower limb wearable device, a bandage-like lower limb wearable device or a sticking lower limb wearable device and etc..

The electronic airbag comprises an airbag body and a gas generator in which substance such as sodium nitride or ammonium nitrate is stored. When the electronic airbag is activated, the gas generator performs a point explosion action so that the substance stored in the gas generator is decomposed rapidly and a large amount of gas is generated and fills the airbag. In one embodiment, the muscle condition monitoring device is connected by signal with the upper limb wearable device or the electronic airbag, and when detecting muscle spasm in lower limb, the muscle condition monitoring device sends an electrical signal to the upper limb wearable device or the electronic airbag, which in response to the electrical signal activates the gas generator to perform a point explosion action, so that the substance such as sodium nitride or ammonium nitrate stored in the gas generator is decomposed rapidly and thus a large amount of non-toxic and unscented gas is generated and fills the airbag which provides certain buoyancy for a swimmer.

When a swimmer wearing the swimming wearable device provided by the embodiments of the present disclosure swims, the muscle condition monitoring device sends a signal to the upper body wearable device or the electronic airbag after detecting muscle spasm, which signal can activate the upper body wearable device or can activate the electronic airbag to open. The airbag in an open state can provide certain buoyancy for the swimmer and protect the swimmer for his or her safety. Therefore, the swimming wearable device according to the embodiments of the present disclosure can reduce drowning accidents and improve safety of swimming exercise.

In one embodiment of the present disclosure, the swimming wearable device comprises at least two lower limb wearable devices. The upper body wearable device comprises at least one upper limb wearable device. Each upper limb wearable device comprises an electronic airbag and the muscle condition monitoring device of each of the at least two lower limb wearable devices is connected by signal with each of the at least one upper limb wearable device or each electronic airbag. And when a muscle condition monitoring device of any one of the at least two lower limb wearable devices detects muscle spasm, the muscle condition monitoring device sends a signal to the at least one upper limb wearable devices or to an electronic airbag of one of the at least one upper limb wearable devices, thereby making the one of the at least two upper limb wearable devices activate its electronic airbag or activating the electronic airbag to open.

The numbers of the upper limb wearable device and the lower limb wearable device are not restricted, but can be one or plural according to the body weight and the body condition of the swimmer. As illustrated in Fig.2, the swimming wearable device comprises two upper limb wearable devices 1 and two lower limb wearable devices 2, wherein:
each upper limb wearable device 1 comprises an electronic airbag 11; each lower limb wearable device 2 comprises a muscle condition monitoring device 21; each muscle condition monitoring device 21 is connected by signal with the two upper limb wearable devices 1 respectively or is connected by signal with the electronic airbags 11 of the two upper limb wearable devices 1 respectively; and when detecting muscle spasm, each muscle condition monitoring device 21 can send a signal to the two upper limb wearable devices 1 or the electronic airbags 11 of the two upper limb wearable devices, so as to make the two upper limb wearable devices 1 activate their electronic airbags 11 to open respectively or to activate the electronic airbags 11 of the two upper limb wearable devices 1.

When a swimmer wearing the swimming wearable device provided by the embodiments of the present disclosure swims, if a spasm in his/her leg or foot occurs, the muscle condition monitoring device 21 can send a signal to the two upper limb wearable devices 1 or the electronic airbags 11 of the two upper limb wearable devices 1, so that the two upper limb wearable devices 1 activate their electronic airbags 11 to open respectively or the electronic airbags 11 are activated to open. The electronic airbags 11 in an open state provide certain buoyancy for the swimmer and protect the swimmer for his or her safety.

The wearing portions of the upper limb wearable device 1 and the lower limb wearable device 2 are not restricted. For example, the upper limb wearable device 1 can be worn at a swimmer's wrist or forearm, and the lower limb wearable device 2 can be worn at a swimmer's ankle or calf. To improve sporting comfort and reduce binding sense, in one embodiment of the present disclosure, the upper limb wearable device 1 is worn at a swimmer's wrist and the lower limb wearable device 2 is worn at a swimmer's ankle.

In one embodiment of the present disclosure, the muscle condition monitoring device of each lower limb wearable device is connected by wireless signal with the electronic airbag of the at least one upper limb wearable device. As illustrated in Fig.2, each muscle condition monitoring device 21 is connected by wireless signal with the electronic airbags 11 of the two upper limb wearable devices 1, for example, by Bluetooth or infrared connection. Connection by wireless signal between the muscle condition monitoring device 21 and the electronic airbag 11 can reduce binding sense of the swimmer and improve his/her comfort.

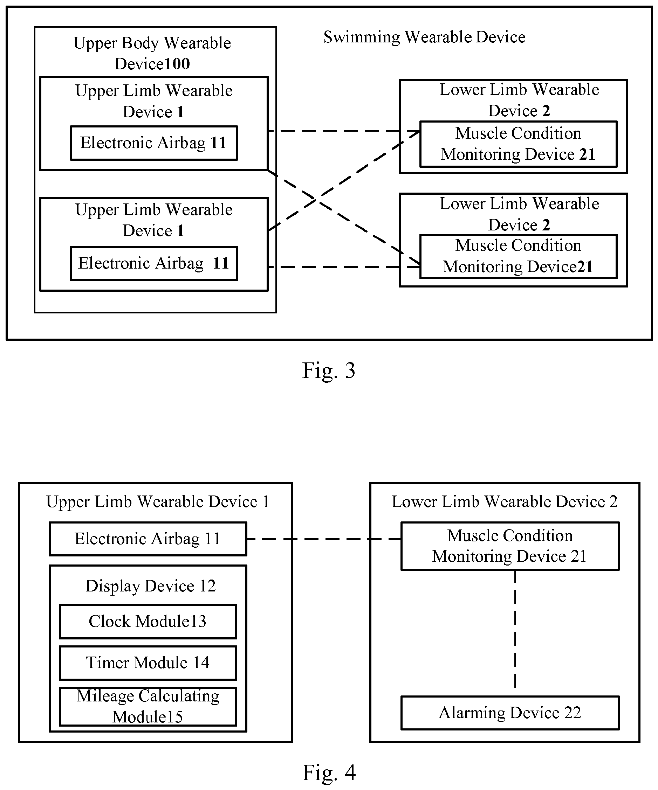

In another embodiment of the present disclosure, the muscle condition monitoring device of each lower limb wearable device is connected by wireless signal with the at least one upper limb wearable device. As illustrated in Fig.3, each muscle condition monitoring device 21 is connected by wireless signal with two upper limb wearable devices 1, for example by Bluetooth or infrared connection. Connection between the muscle condition monitoring device 21 and the upper limb wearable device 1 by wireless signal can reduce binding sense of the swimmer and improve his/her comfort.

As illustrated in Fig.4, to further improve the safety of swimming exercise, the lower limb wearable device 2 further comprises an alarming device 22 connected by signal with the muscle condition monitoring device 21. And when detecting muscle spasm, the muscle condition monitoring device 22 can activate the alarming device 22 to give an alarm. The alarming device 22 provides an alarm signal notifying the abnormality of the swimmer to others or a lifeguard, thereby reducing drowning accidents and further improving safety of swimming exercise. The type of the alarming device 22 is not restricted, but can be a sound alarm or an audible and visual alarm and the like, for example.

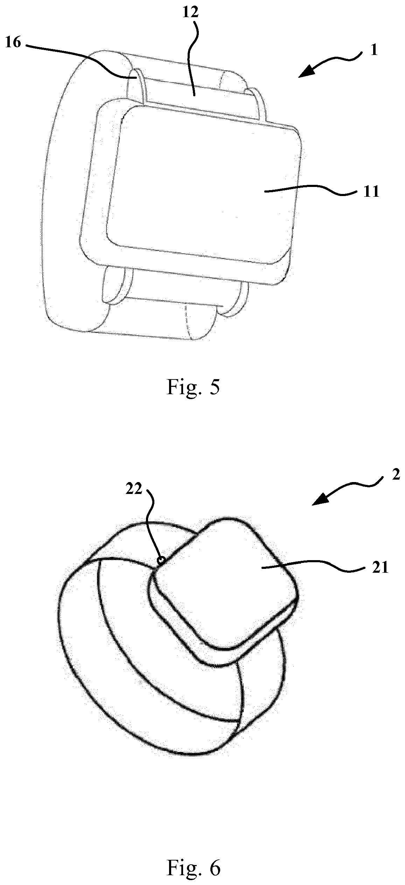

The structural forms of the upper limb wearable device 1 and the lower limb wearable device 2 are not restricted. For example, the upper limb wearable device 1 and the lower limb wearable device 2 can be of sticking type in which they are stuck and affixed to the upper and lower limbs of the swimmer respectively. As illustrated in Fig.5 and Fig.6, in the embodiment, to have the wearable device firmly affixed, the upper limb wearable device 1 is of arm wearing type, the lower limb wearable device 2 is of ankle wearing type, and the upper limb wearable device 1 and the lower limb wearable device 2 are ring-like as a whole and can enclose the swimmer's arm and ankle respectively and be affixed thereto.

In the embodiment as illustrated in Fig.4, the upper limb wearable device 1 further comprises a display device 12. One of a clock module 13, a timer module 14 and a mileage calculating module 15 can be integrated in the display device 12 so that the upper limb wearable device 1 can have a function of time displaying, time calculating or swimming mileage calculating and etc.. A swimmer can be aware of sport information conveniently and timely by observing display information of the display device 12. For example, the mileage calculating module 15 can be a GPS mileage calculating module or a vibration counting mileage calculating module.

As illustrated in Fig.5, in the upper limb wearable device 1, the display device 12 is disposed on one side of the electronic airbag 11. The display device 12 and the electronic airbag 11 are disposed on a structural connection member 16 which serves a function of connecting, supporting and fixing. When being worn, the display device 12 and the electronic airbag 11 are disposed towards an outer side of an arm. A swimmer can learn his/her own sport information conveniently by observing the display device 12. Once a spasm in leg or foot occurs, the electronic airbag 11 can be timely popped up at the outer side of the arm so as to protect the safety of the swimmer.

Based on the same inventive concept, at least one embodiment of the present disclosure further provides an upper limb wearable device which comprises an electronic airbag. The muscle condition monitoring device of the lower limb wearable device is connected by signal with the upper limb wearable device or the electronic airbag of the upper limb wearable device. And when the muscle condition monitoring device detects muscle spasm, the muscle condition monitoring device sends a signal to the upper limb wearable device or the electronic airbag of the upper limb wearable device so as to make the upper limb wearable device activate the electronic airbag or activate the electronic airbag to open.

In one embodiment of the present disclosure, the upper limb wearable device comprises a display device in which at least one of a clock module, a timer module and a mileage calculating module is integrated.

Drowning accidents can be reduced and safety of swimming exercise can be improved by wearing the lower limb wearable device comprising the muscle condition monitoring device and the upper limb wearable device provided by the embodiments of the present disclosure.

It can be appreciated that in the various embodiments of the present disclosure, all the electronic components in the upper body wearable device and the lower limb wearable device should be designed in a waterproof manner. Alternatively, the upper body wearable device and the lower limb wearable device should adopt a waterproof design as a whole respectively.

The foregoing are merely exemplary embodiments of the disclosure, but are not used to limit the protection scope of the disclosure. The protection scope of the disclosure shall be defined by the attached claims.

## Claims

1. A swimming wearable device, comprising an upper body wearable device (100), wherein the upper body wearable device (100) comprises an electronic airbag (11);
**characterized in that**: the swimming wearable device further comprises a lower limb wearable device (2), wherein the lower limb wearable device (2) comprises a muscle condition monitoring device (21); the muscle condition monitoring device (21) is connected by signal with the upper body wearable device (100) or with the electronic airbag (11) of the upper body wearable device (100), and when detecting a muscle spasm in lower limb, the muscle condition monitoring device (21) sends a signal to the upper body wearable device (100) or the electronic airbag (11) of the upper body wearable device (100), so as to make the upper body wearable device (100) activate the electronic airbag (11) to open or to activate the electronic airbag (11) to open.

2. The swimming wearable device according to claim 1, wherein
the upper body wearable device (100) comprises a vest-like upper body wearable device, a bandage-like upper body wearable device, a sticking upper body wearable device or an arm wearing upper body wearable device.

3. The swimming wearable device according to claim 1 or 2, wherein
the lower limb wearable device (2) comprises an ankle wearing lower limb wearable device, a bandage-like lower limb wearable device or a sticking lower limb wearable device.

4. The swimming wearable device according to any one of claims 1 to 3, comprising at least two lower limb wearable devices (2), the upper body wearable device (100) comprising at least one upper limb wearable devices (1), wherein
each upper limb wearable device (1) comprises an electronic airbag (11) and the muscle condition monitoring device (21) of each of the at least two lower limb wearable devices (2) is connected by signal with each of the at least one upper limb wearable device (1) or the electronic airbag (11) of one of the at least one upper limb wearable device (1), and when detecting a muscle spasm, the muscle condition monitoring device (21) sends a signal to one of the at least one upper limb wearable devices (1) or to the electronic airbag (11) of one of the at least two upper limb wearable devices (1), so as to make the one of the at least one upper limb wearable devices (1) activate the electronic airbag (11) to open or to activate the electronic airbag (11) to open.

5. The swimming wearable device according to any one of claims 1 to 4, wherein the lower limb wearable device (2) comprises an alarming device (22) which is connected by signal with the muscle condition monitoring device (21), and when detecting a muscle spasm, the muscle condition monitoring device (21) activates the alarming device (22) to give an alarm.

6. The swimming wearable device according to any one of claims 4 to 5, wherein the muscle condition monitoring device (21) of each lower limb wearable device (2) is connected by wireless signal with the at least one upper limb wearable device (1) or with the electronic airbag (11) of the at least one upper limb wearable device (1).

7. The swimming wearable device according to any one of claims 4 to 6, wherein the upper limb wearable device (1) further comprises a display device (12).

8. The swimming wearable device according to claim 7, wherein at least one of a clock module (13), a timer module (14) and a mileage calculating module (15) is integrated in the display device (12).

9. The swimming wearable device according to claim 8, wherein the mileage calculating module (15) comprises a GPS mileage calculating module or a vibration counting mileage calculating module.

10. The swimming wearable device according to any one of claims 1 to 9, wherein the electronic airbag (11) comprises a gas generator in which substance that can be rapidly decomposed to generate gas is stored.

## Patentansprüche

1. Tragbare Vorrichtung zum Schwimmen, umfassend eine tragbare Vorrichtung (100) für Oberkörper, wobei die tragbare Vorrichtung (100) für Oberkörper einen elektronischen Airbag (11) umfasst;
**dadurch gekennzeichnet: dass** die tragbare Vorrichtung zum Schwimmen außerdem eine tragbare Vorrichtung (2) für untere Extremitäten umfasst, wobei die tragbare Vorrichtung (2) für untere Extremitäten eine Muskelzustands-Überwachungsvorrichtung (21) umfasst; wobei die Muskelzustands-Überwachungsvorrichtung (21) mittels Signal mit der tragbaren Vorrichtung (100) für Oberkörper oder mit dem elektronischen Airbag (11) der tragbaren Vorrichtung (100) für Oberkörper verbunden ist, und wobei die Muskelzustands-Überwachungsvorrichtung (21) während der Detektion eines Muskelkrampfes in einer unteren Extremität ein Signal an die tragbare Vorrichtung (100) für Oberkörper oder den elektronischen Airbag (11) der tragbaren Vorrichtung (100) für Oberkörper sendet, um die tragbare Vorrichtung (100) für Oberkörper zu aktivieren, den elektronischen Airbag (11) zu öffnen, oder um den elektronischen Airbag (11) zu aktivieren zum Öffnen.

2. Tragbare Vorrichtung zum Schwimmen gemäß Anspruch 1, wobei die tragbare Vorrichtung (100) für Oberkörper eine westenartige tragbare Vorrichtung für Oberkörper, eine bandagenartige tragbare Vorrichtung für Oberkörper, eine anhaftende tragbare Vorrichtung für Oberkörper oder eine tragbare Armtrage-Vorrichtung für Oberkörper umfasst.

3. Tragbare Vorrichtung zum Schwimmen gemäß Anspruch 1 oder 2, wobei die tragbare Vorrichtung (2) für untere Extremitäten eine tragbare Fußgelenktrage-Vorrichtung für untere Extremitäten, eine bandagenartige tragbare Vorrichtung für untere Extremitäten oder eine anhaftende tragbare Vorrichtung für untere Extremitäten umfasst.

4. Tragbare Vorrichtung zum Schwimmen gemäß einem der Ansprüche 1 bis 3, umfassend zumindest zwei tragbare Vorrichtungen (2) für untere Extremitäten, wobei die tragbare Vorrichtung (100) für Oberkörper zumindest eine tragbare Vorrichtung (1) für obere Extremitäten umfasst, wobei jede tragbare Vorrichtung (1) für obere Extremitäten einen elektronischen Airbag (11) umfasst, und wobei die Muskelzustands-Überwachungsvorrichtung (21) von jeder der zumindest zwei tragbaren Vorrichtungen (2) für untere Extremitäten mittels Signal mit jeder von der zumindest einen tragbaren Vorrichtung (1) für obere Extremitäten oder dem elektronischen Airbag (11) von einer von der zumindest einen tragbaren Vorrichtung (1) für obere Extremitäten verbunden ist, und wobei die Muskelzustands-Überwachungsvorrichtung (21), wenn ein Muskelkrampf detektiert wird, ein Signal sendet an eine von der zumindest einen tragbaren Vorrichtung (1) für obere Extremitäten oder den elektronischen Airbag (11) von einer von den zumindest zwei tragbaren Vorrichtungen (1) für obere Extremitäten, um die eine von der zumindest einen tragbaren Vorrichtung (1) für obere Extremitäten zu aktivieren, den elektronischen Airbag (11) zu öffnen, oder um den elektronischen Airbag (11) zu aktivieren, zu öffnen.

5. Tragbare Vorrichtung zum Schwimmen gemäß einem der Ansprüche 1 bis 4, wobei die tragbare Vorrichtung (2) für obere Extremitäten eine Alarmierungsvorrichtung (22) umfasst, die mittels Signal mit der Muskelzustands-Überwachungsvorrichtung (21) verbunden ist, und wobei die Muskelzustands-Überwachungsvorrichtung (21), wenn ein Muskelkrampf detektiert wird, die Alarmierungsvorrichtung (22) aktiviert, um einen Alarm zu geben.

6. Tragbare Vorrichtung zum Schwimmen gemäß einem der Ansprüche 4 bis 5, wobei die Muskelzustands-Überwachungsvorrichtung (21) von jeder tragbaren Vorrichtung (2) für untere Extremitäten mittels drahtlosem Signal verbunden ist mit der zumindest einen tragbaren Vorrichtung (1) für obere Extremitäten oder mit dem elektronischen Airbag (11) von der zumindest einen tragbaren Vorrichtung (1) für obere Extremitäten.

7. Tragbare Vorrichtung zum Schwimmen gemäß einem der Ansprüche 4 bis 6, wobei die tragbare Vorrichtung (1) für obere Extremitäten außerdem eine Anzeigevorrichtung (12) umfasst.

8. Tragbare Vorrichtung zum Schwimmen gemäß Anspruch 7, wobei in die Anzeigevorrichtung (12) zumindest eines von einem Uhrmodul (13), einem Timer-Modul (14) und einem Kilometerstandberechnungsmodul (15) integriert ist.

9. Tragbare Vorrichtung zum Schwimmen gemäß Anspruch 8, wobei das Kilometerstands-Berechnungsmodul (15) ein GPS-Kilometerstands-Berechnungsmodul oder ein Vibrationszähl-Kilometerstandsberechnungsmodul umfasst.

10. Tragbare Vorrichtung zum Schwimmen gemäß einem der Ansprüche 1 bis 9, wobei der elektronische Airbag (11) einen Gasgenerator umfasst, in welchem Substanz gespeichert ist, die zur Erzeugung von Gas schnell abgebaut werden kann.

## Revendications

1. Dispositif portable de natation comprenant un dispositif portable corporel supérieur (100), dans lequel le dispositif portable corporel supérieur (100) comprend un coussin d'air électronique (11) ;
**caractérisé en ce que** : le dispositif portable de natation comprend en outre un dispositif portable de membre inférieur (2), dans lequel le dispositif portable de membre inférieur (2) comprend un dispositif de surveillance de l'état musculaire (21) ; le dispositif de surveillance de l'état musculaire (21) est connecté par signal au dispositif portable corporel supérieur (100) ou au coussin d'air électronique (11) du dispositif portable corporel supérieur (100), et lorsqu'il détecte un spasme musculaire dans le membre inférieur, le dispositif de surveillance de l'état musculaire (21) envoie un signal au dispositif portable corporel supérieur (100) ou au coussin d'air électronique (11) du dispositif portable corporel supérieur (100), de façon à ce que le dispositif portable corporel supérieur (100) active le coussin d'air électronique (11) pour ouvrir ou pour activer l'ouverture du coussin d'air électronique (11) .

2. Dispositif portable de natation selon la revendication 1, dans lequel
le dispositif portable corporel supérieur (100) comprend un dispositif portable corporel supérieur de type veste, un dispositif portable corporel supérieur de type bandage, un dispositif portable corporel supérieur collant ou un dispositif portable corporel supérieur de type se portant sur le bras.

3. Dispositif portable de natation selon la revendication 1 ou 2, dans lequel
le dispositif portable de membre inférieur (2) comprend un dispositif portable de membre inférieur se portant à la cheville, un dispositif portable de membre inférieur de type bandage ou un dispositif portable de membre inférieur collant.

4. Dispositif portable de natation selon l'une quelconque des revendications 1 à 3, comprenant au moins deux dispositifs portables de membre inférieur (2), le dispositif portable corporel supérieur (100) comprenant au moins un dispositif portable de membre supérieur (1), dans lequel
chaque dispositif portable de membre supérieur (1) comprend un coussin d'air électronique (11) et le dispositif de surveillance de l'état musculaire (21) de chacun des au moins deux dispositifs portables de membre inférieur (2) est connecté par signal à chacun de l'au moins un dispositif portable de membre supérieur (1) ou au coussin d'air électronique (11) de l'un de l'au moins un dispositif portable de membre supérieur (1), et lorsqu'il détecte un spasme musculaire, le dispositif de surveillance de l'état musculaire (21) envoie un signal à un de l'au moins un dispositif portable de membre supérieur (1) ou au coussin d'air électronique (11) de l'un des aux moins deux dispositifs portables de membre supérieur (1), de façon à ce que l'un des au moins un dispositif portable de membre supérieur (1) active le coussin d'air électronique (11) pour ouvrir ou pour activer l'ouverture du coussin d'air électronique (11).

5. Dispositif portable de natation selon l'une quelconque des revendications 1 à 4, dans lequel le dispositif portable de membre inférieur (2) comprend un dispositif d'alarme (22) qui est connecté par signal au dispositif de surveillance de l'état musculaire (21), et lorsqu'il détecte un spasme musculaire, le dispositif de surveillance de l'état musculaire (21) active le dispositif d'alarme (22) pour donner une alarme.

6. Dispositif portable de natation selon l'une quelconque des revendications 4 à 5, dans lequel le dispositif de surveillance de l'état musculaire (21) de chaque dispositif portable de membre inférieur (2) est connecté par signal sans fil à l'au moins un dispositif portable de membre supérieur (1) ou au coussin d'air électronique (11) de l'au moins un dispositif portable de membre supérieur (1).

7. Dispositif portable de natation selon l'une quelconque des revendications 4 à 6, dans lequel le dispositif portable de membre supérieur (1) comprend en outre un dispositif d'affichage (12).

8. Dispositif portable de natation selon la revendication 7, dans lequel au moins un d'un module d'horloge (13), d'un module de minuterie (14) et d'un module de calcul de kilométrage (15) est intégré dans le dispositif d'affichage (12).

9. Dispositif portable de natation selon la revendication 8, dans lequel le module de calcul de kilométrage (15) comprend un module de calcul de kilométrage par GPS ou un module de calcul de kilométrage par comptage des vibrations.

10. Dispositif portable de natation selon l'une quelconque des revendications 1 à 9, dans lequel le coussin d'air électronique (11) comprend un générateur de gaz dans lequel est stockée une substance qui peut être rapidement décomposée pour générer du gaz.
